(19) Europäisches Patentamt
European Patent Office
Office européen des brevets

(11) **EP 3 892 716 A1**

(12) **EUROPEAN PATENT APPLICATION**

(43) Date of publication:
**13.10.2021 Bulletin 2021/41**

(51) Int Cl.:
*C12N 1/20* (2006.01)     *C12N 1/04* (2006.01)
*A01N 63/20* (2020.01)     *A01N 63/22* (2020.01)
*C05F 11/08* (2006.01)     *C12R 1/065* (2006.01)
*C12R 1/11* (2006.01)

(21) Application number: **20168415.6**

(22) Date of filing: **07.04.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Biofor System d.o.o.**
**11080 Zemun (RS)**

(72) Inventors:
• **Raicevic, Vera**
  **11070 Belgrade (RS)**
• **Jovicic-Petrovic, Jelena**
  **11030 Belgrade (RS)**
• **Levic, Steva**
  **11080 Belgrade (RS)**
• **Nedovic, Viktor**
  **11070 Belgrade (RS)**

(74) Representative: **Patentni Biro AF d.o.o.**
**Kotnikova 32, p.p. 2706**
**1001 Ljubljana (SI)**

(54) **NOVEL PLANT GROWTH PROMOTING BACTERIAL STRAINS, AN IMPROVED METHOD FOR ENCAPSULATION OF BACTERIA AND A BIOFERTILIZER COMPRISING ENCAPSULATED BACTERIA PREPARED BY THE SAID METHOD**

(57)     The present invention belongs to the field of agriculture, more particularly to the field of organic fertilizers which contain cultures of live and active microorganisms. The invention relates to novel bacterial strains simultaneously exhibiting production of phosphatases for mineralization of organic phosphorus compounds, and phosphorus solubilisation for solubilizing insoluble phosphorus salts. Further, the invention also relates to an improved method for encapsulation of bacteria and a bio fertilizer comprising encapsulated bacteria prepared by the said process.

Sodium alginate, calcium chloride, starch and optionally a colouring are used as external encapsulation media, wherein the bacteria are added to the encapsulation mixture with their whole microbial media (cells plus media containing unprocessed components and microbial products). This is essential, since microbial products have stimulus effects on plants while residual components of medium can bust microbial activity and help microbial culture activation. A further characteristic of the process is slow drying of encapsulates at mild temperatures, which results in evaporated water and further cell stabilization.

**Description**

**Field of the invention**

The present invention belongs to the field of agriculture, more particularly to the field of soil treatment intended to increase the product yield. In addition, this invention also belongs to the field of fertilisers, in particular to the field of organic fertilisers which contain cultures of live and active microorganisms. The invention relates to novel bacterial strains simultaneously exhibiting production of phosphatases for mineralization of organic phosphorus compounds, and phosphorus solubilisation for solubilizing insoluble phosphorus salts. Further, the invention also relates to an improved method for encapsulation of bacteria and a biofertilizer comprising encapsulated bacteria prepared by the said process.

**Background of the invention and the technical problem**

Phosphorus (P) is an important nutrient in plant nutrition. In terms of quantitative requirements for plant nutrients, phosphorus is the second most important element, after nitrogen. It is present in the soil in organic and mineral compounds, but its availability is limited because it occurs mainly in insoluble forms. The phosphorus content in the average soil is about 0.05% (w/w), but only 0.1% is available for plants due to poor solubility and soil fixation. In order to fulfil the need of available phosphorus for plants and also to meet the ever-growing global food demand, conventional mineral phosphorus fertilizers are used. The application of these fertilizers has many negative impacts on the environment, total soil health and accumulation of toxic elements such as high concentration of selenium (Se), arsenic (As), unfavourable carbon footprint, eutrophication and degradation of terrestrial, freshwater and marine ecosystems. In case of applied phosphorus in the form of mineral fertilizers, plants can use only a small amount, because 75-90% of the added phosphorus precipitates by metal-cation complexes and quickly becomes fixed in the soil. Thus, the soil conditions dictate the availability degree of phosphorus compounds.

This increases the concern about the environment quality and points out the need to develop sustainable crop production and the green technology implementation. The use of soil bacteria from group of Phosphate Solubilizing Microorganisms (also called PSM) plays a significant role in sustainable management with limited phosphate resources, which is in line with sustainable development strategies and green technologies. Microbial strains are capable of solubilizing/mineralization of insoluble phosphates, which in turn becomes available to plants. These microorganisms improve the growth and yield of a wide range of crops and are considered to be the best ecological tools for improving the supply of phosphorus. Microorganisms that solubilize phosphorus (PSM) represent a promising strategy to increase food production in the world without causing environmental hazards.

PSM are capable to enable better plant supply with phosphorus and thus increase the crop quality and yield. The main problem in the biofertilizer commercialization is, however, poor bacterial survival during storage. Therefore the development of formulations based on encapsulation of bacterial cells is essential, because encapsulation will protect the active components, improve the sustainability as well as stability during storage and handling. Also, the method of encapsulation and use of the adequate carrier may influence bacterial survival, thus affecting the final plant supply with phosphorus. Hence there is a need for improved encapsulation methods of bacterial strains effective in procedures for increasing phosphorus availability for plants. Besides that, the adequate encapsulation technique should be in accordance with agronomic practice. Even though encapsulation has been widely used in food processing and pharmaceutical products, its use in soil biofertilization has mostly been neglected.

The goal of this invention is to provide useful bacterial strains to increase phosphorus availability in agricultural soil and to provide a suitable encapsulation method, which will enable preparation of effective biofertilizer compositions. The technical problem is development of an improved method for encapsulation of bacteria, especially phosphomobilizing bacterial strains, which will allow preparation of a biofertilizer that will be effective, easy to use and environmentally friendly.

**State of the art**

Many bacterial strains are known to influence plant growth, wherein patent applications and patents CN108753654, FR2806420, CN105967856, CN110438036, RU2008129928, EP2227931 and SU1703634 disclose use of *Azotobacter chroococcum* in biofertilizers. Document FR2806420 also mentions that *Bacillus megaterium* is used with the *A. chroococcum.* Apart from these strains also other bacteria affect plant growth and they are well-known in the art.

As already mentioned above, bacteria to be used in biofertilizers have to be suitably processed, in order to be stable and active for prolonged periods. Encapsulation is one of such methods and it has been well-known in the field of probiotic bacteria. For example, Zanjani et al. (2014, Iran J Pharm Res. 13(3): 843-852) describe microencapsulation of probiotic bacteria with alginate-gelatinized starch coated with chitosan.

Etchepare et al. (2015; doi: 10.1590/0103-8478cr20140938) in their article review methods for encapsulation of probiotic bacteria using calcium alginate, one is extrusion method by dripping a solution of sodium alginate into a solution of a calcium salt, leading to the phenomenon of external ionic gelation. The addition of other adjuvant materials such as polymers (fructooligosaccharides, inulin and resistant starches) improves the viability of bacteria and makes the encapsulation more efficient.

Huq et al. (2013; doi: 10.1080/10408398.2011.573152) in their review article discuss improving viability of the probiotic bacteria with several types of biopolymers such as alginate, chitosan, gelatine, whey protein isolate, cellulose derivatives used for encapsulation. Several methods of encapsulation such as spray drying, extrusion and emulsion are also discussed.

Although the encapsulation of plant-growth promoting microorganisms has mostly been neglected, there are few available documents relating to this issue. Namely, patent application CN102765983 describes an organic and inorganic compound fertilizer, which is characterized by being prepared by the steps of: adding chemical fertilizer into an organic raw material, mixing and then granulating by adopting opposite roller extrusion at low temperature (60-65 °C), spraying slurry to encapsulate the granules by amino acid; and implanting a microbial agent to the encapsulation film (6-8 hundred million of bacteria per gram), wherein the microbial agent comprises plant growth promoting bacteria, bacilli and actinomycetes.

Document CN104961591 discloses a controlled-released efficacy-improved biological fertilizer and a preparation method thereof. The fertilizer comprises a nitrogen, phosphorus and potassium compound fertilizer, microelements and a compound additive; the compound additive comprises a nitrification inhibitor, a urease inhibitor, phosphate solubilizing bacteria and humid soil.

Document CN109206204 discloses an antibacterial and insecticidal sustained release compound fertilizer and a preparation method thereof. The antibacterial and insecticidal sustained release compound fertilizer comprises the following raw materials by weight: 160-220 parts of an encapsulation material, 200-450 parts of fertilizer granules, 100-200 parts of an antibacterial and insecticidal composition, 20-30 parts of waste residue, and 15-25 parts of pomacea canaliculata powder. The fertilizer is prepared by granulation, drying, cooling and encapsulation.

Document CN101244957 describes a combined fertilizer comprising organic fertilizers, inorganic fertilizers and microorganism fertilizers to form granular by adopting multigrade and layered granulating technique, and then an encapsulation is used for covering a layer of a natural polymer adhesive film on the surface of the granular, thus achieving the aim of controlling applying nutrients for crops according to regulations of fertilizer needs of crops.

These disclosures are not similar to the present invention as the medium used for encapsulation is different. Furthermore, the traits performed by the plant-growth promoting bacteria used in the biofertilizer according to the invention differ from the known bacterial strains.

**Description of the solution of the technical problem**

The present invention relates to plant-growth promoting bacteria simultaneously exhibiting the ability to solubilize insoluble phosphorus salts and mineralize organic phosphorus compounds, which has never been reported before. Further, the invention relates to an improved method of encapsulation and well as preparation of biofertilizers to be used in agriculture. Finally, the invention also relates to the biofertilizer and its use. The technical problem is solved according to the independent claims, while preferred embodiments are presented in dependent claims.

The development of the invention began with isolation of environmental bacterial, which were found to be able to solubilize phosphate. The essence of the invention is two bacterial strains, which were characterized and identified by molecular methods (16S DNA) as *Azotobacter chroococcum* and *Bacillus megaterium*. Bacterial strains were isolated from agricultural soil using standard dilution method and cultivation on two selective media. The NBRIP medium (National Botanical Research Institute's Phosphate) was used to search for isolates forming halo zones caused by phosphate solubilization. After purification of phosphate solubilizing strains, all of the isolates were tested for phosphatase production by API ZYM test (Biomerieux, France), and after that procedure, strain belonging to *Bacillus* was selected as one exibiting both mechanisms involved in increase of phosphate availability in soil. In order to select nitrogen fixing strain included in phosphate solubilization, diazotrophs were izolated using selective Fidorov's medium and, after purification, screened for their capability for phosphate solubilisation by applying spot techniques on NBRIP medium. After two-week incubation, halo zones and colony diameter were measured. The selected strain belonging to *Azotobacter* genus showed the strongest possibility to solubilise P. All these procedures were done according to standard practice and protocols known by those skilled in the art. After identification, active strains were deposited in BCCM Belgian Coordinated Collections of Microorganisms, BCCM/LGM Bacteria Collection, (deposit date 28/05/2019) in the name of the Applicant Biofor System d.o.o. under the following identification (accession numbers):

- *A. chroococcum* F14/2 = LMG P-31444, and
- *B. megaterium* 11/3 = LMG P-31445.

The said strains were tested for their ability to transform/dissolve stable phosphorus chemical forms into phosphorus available for plant uptake via root system. Index of phosphorus solubilisation is a test for checking solubilisation of insoluble phosphorus salts and has been determined by spot technique on NBRIP medium. Index of P solubilisation was calculated as:

$$SI=[(colony\ diamteter + halo\ zone\ diameter)/colony\ diameter]*100$$

and it has been determined that SI was 1.11 and 1.94 for *B. megaterium* and *A. chroococcum,* respectively.

The enzymatic profiles of both strains were determined by API ZYM (Biomerieux, France), using manufacturer's instructions. Both strains produce acid phosphatases and alkaline phosphatases, which is an indication for ability to mineralize organic phosphorus compounds.

Thus, it can be concluded that chosen bacterial strains manifest both mechanisms important for phosphorus availability - solubilization of insoluble phosphorus salts as well as mineralization of organic phosphorus compounds. These two activities have never been observed to occur together. Besides these characteristics, both strains are characterized as producers of exopolysaccharides important for plant root colonization, and *A. chroococcum* additionally improves nitrogen availability, since it is known as nitrogen assimilating microorganism.

The described microorganisms are due to their traits suitable to be used in biological fertilizers according to the invention. However, other known bacterial strains known for their positive effect on plant growth may be used in the fertilizer as described below. For example, the biofertilizer may comprise other bacterial strains belonging to genus *Bacillus, Pseudomonas* and *Azotobacter.*

The fertilizer according to the invention is prepared by encapsulation of plant-growth promoting microbes. The aim of encapsulation is to obtain a dried encapsulated formulation that will provide prolonged storage, while it will at the same time maintain high cell viability and will enable user friendly application in the field conditions. The process comprises the following steps:

a) growth of at least one of the plant-growth promoting bacterial strain in a suitable medium,
b) mixing of the whole medium from step a) with alginate, starch and optionally a colorant,
c) dropping the mixture from step b) into calcium chloride preferably using a nozzle with 8 holes, while encapsulation mixture is pumped using mechanical pump; while other techniques such as vibration technique, electrostatic extrusion, and dispersion with compressed gas, can be used;
d) incubation of the mixture obtained in step c) to allow gelation, preferably for 15 to 30 minutes, more preferably for about 15 min, to obtain encapsulates;
e) optionally rinsing the encapsulates obtained in step d) with water to remove surface components,
f) spreading the rinsed encapsulates onto a suitable surface such as a drying pan and drying at temperature at least 30 °C for at least 2 days or less if the temperature is higher; wherein the preferred drying is preferably in an oven at 37 °C for 1 to 2 days (depending on applied bacterial strain and encapsulate size).

The bacterial strain for encapsulation may be one or more. If two or more, they can be grown and/or encapsulated together. If their characteristics and growth conditions are similar, they can be grown and encapsulated together. If their growth requirements differ, they can be grown separately and encapsulated together. Another option is to grow and encapsulate individual strains separately and then combine the encapsulated strains. In this way, the invention enables subsequent combining of different strains depending on specific purpose, environmental conditions, etc., and thus allows a huge variability of use.

Specifically, the *A. chroococcum* and *B. megaterium* are grown separately, wherein the first is preferably grown in peptone, dextrose and NaCl comprising medium, while the latter is preferably grown in tryptone soya broth. Both strains are incubated with mixing on a rotary shaker, for example at 150 rpm, and at temperature around 30 °C for around 48 h to 72 h, wherein these conditions may be varied. These strains may be used in the biofertilizer alone, together or in combination with any other bacterial species beneficial for plants. In such microbial communities, only one or both said bacterial strains may be present.

The preferred ratios in step b) of the method described above are:

- at least 1:1, preferably 2:1 liquid bacterial culture : alginate solution; wherein the alginate solution is preferably 3% (w/w);
- 5 to 15 g, preferably 10g of starch per 100ml of liquid bacterial culture depending on required size of encapsulates (i.e. increased starch content provide larger encapsulates);
- And the amount of the optional colorant to yield yellow coloured encapsulates is 0.2g riboflavin/100ml of the encapsulation mixture.

Suitable colorants are food-grade colorants such as riboflavin. Their presence ensures that the granules are coloured, so that fertilizers with different microorganisms can be easily distinguished. Also, color of encapsulates with starch is white which also help their identification and help identification on the ground in the case of surface application.

The above-described method offers the possibility to apply other encapsulation techniques with the aim to regulate the particle size. Some of those are vibration technique, electrostatic extrusion, and dispersion with compressed gas. The size of encapsulates is approximately 3 to 5 mm and are approximately spherical in shape.

Rinsing and drying may be performed in any suitable way known in the field. Low water content, preferably far below 10%, ensures prolonged stability. Also, storage stability is ensured by slow drying of encapsulates. After drying encapsulates are ready for packaging and/or use.

In comparison to known methods, the present invention uses the whole medium as active ingredient, i.e. cells plus medium's components and microbial metabolic products. This is essential, since microbial products have stimulus effects on plants while residual components of medium can bust microbial activity and help microbial culture activation. The encapsulates thus comprise components of cells and bacterial growth medium and/or cell metabolites and molecules present in used growth medium, which differs from presently known solutions.

Activity of bacteria is ensured by mild conditions of encapsulation process, as alginate and calcium chloride are both friendly towards the tested microbes under the described concentrations. A further advantage of the method according to the invention is slow drying of encapsulates, which reduce water content and help cells stabilization. Also, slow drying most probably help cells to overcome stress.

The method is simple and includes only compounds that don't contaminate soil or water, which makes it cheap (contrary to e.g. freeze drying or spray drying) and environmentally friendly as it is based on ecological friendly materials and doesn't use any organic solvent or harmful chemical.

Furthermore, the process allows preparation of various fertilizers comprising one or more bacterial species, wherein the fertilizer may be optimized for incorporation into the soil via available fertilizer spreaders. Said fertilizers may be used for promoting growth of different plants, primarily crops and fruits, but it can be used in vegetable production as well. Also, encapsulated forms can be used during seeding since they possess satisfactory mechanical strength for incorporation into the soil via fertilizer depositors. The active microorganisms exhibit a good survival performance during encapsulation process as well as after two-month-period of storage at room temperature. In addition, granules are characterized by slow dissolution in soil as both, alginate and starch, are biodegradable, thus providing gradual release of active bacteria and better protection in the soil environment.

The invention will be further described based on possible embodiments and examples.

**Example 1: Isolation of *A. chroococcum* and *B. megaterium***

1.1 Method of isolation and selection of suitable strains

The essence of the invention is two bacterial strains, which were characterized and identified by usual molecular methods using 16S DNA as *Azotobacter chroococcum* and *Bacillus megaterium.* Both bacterial strains were isolated from agricultural soil using standard dilution method and cultivation on two selective media; NBRIP medium was used to search for isolates forming halo zones caused by phosphate solubilization, and Fidorov's medium for nitrogen fixing strains. All strains were screened and after two-week incubation on NBRIP medium, halo zones and colony diameter were measured.

After purification of phosphate solubilizing strains, all of the isolates were tested for phosphatase production by API ZYM test (Biomerieux, France) according to manufacturer's instructions.

Two strains exhibited strong phosphorus solubilization activity, wherein one strain had also fixed nitrogen. In order to identify both strains, standard identification using 16S rRNA has been used.

1.2 Strain identification

DNA isolation was performed by Soil Mini Prep Kit (Zymo Research). After determination of DNA quantity and quality, 16s rRNA region was amplified. *Azotobacter* F14/2 isolate was identified by amplification with primers 63 F and 1387 R. PCR reaction was done in 50 $\mu$l - volime mixture composed of:

- 5 $\mu$l 10xBuffer,
- 1 $\mu$l dNTP mix,
- 2 $\mu$l of each primer,
- 0.2 $\mu$l Taq polimerase,
- 1 $\mu$l template DNA and
- RNA free water up to 50 $\mu$l.

Applied PCR program was: 95°C/5 min, 35 cycles (95°C/30s, 55°C/30s and 72°C/1min), 72°C/1 min.

The isolate belonging to *Bacillus* genus was identified using primer pair UNI 16SR/UNI 16SF to amplify 16s RNA region. Fast Gene Taq HotStart PCR kit (Kappa biosistem) was used for mix preparation. PCR reaction mixture consisted of:

- 10 μl 10xBuffer,
- 3 μl 25 MgCl₂,
- 1 μl dNTP mix,
- 2 μl of each primer,
- 0.2 μl Taq polimerase,
- 1 μl DNK and
- RNA free water until 50 μl.

The following PCR prigram was applied: 95°C/5 min, 35 cycles (95°C/30s, 55°C/30s and 72°/1 min) and 72°C/1 min.

After visualisation of PCR products by gel electrophoresis, the products were sent to sequencing (Macrogen, South Korea). Sequencing was performed in both directions, and cosensus sequences are analysed and compared with representative sequences from Gen Bang database. Sequences of strains 11/3 and F14/2 showed high percentage of identity with sequences of *Bacillus megaterium* and *Azotobacter chroococcum* species from database (97% identity in both cases).

After identification, active strains were deposited in BCCM Belgian Coordinated Collections of Microorganisms, BCCM/LGM Bacteria Collection, (deposit date 28/05/2019), under the following identification:

*A. chroococcum* F14/2 = LMG P-31444, and
*B. megaterium* 11/3 = LMG P-31445.

1.3 Determination of P index and phosphatases

Index of phosphorus solubilisation is a test for checking solubilisation of insoluble phosphorus salts and the index has been determined by spot technique on NBRIP medium. Index of P solubilisation was calculated as:

$$SI=[(colony\ diamteter + halo\ zone\ diameter)/colony\ diameter]*100$$

and it has been determined that SI was 1.11 and 1.94 for *B. megaterium* and *A. chroococcum,* respectively.

The enzymatic profiles of both strains were determined by API ZYM (Biomerieux, France), using manufacturer's instructions. Both strains produce acid phosphatases and alkaline phosphatases, which is an indication for ability to mineralize organic phosphorus compounds.

**Example 2: Preparation of encapsulated bacteria**

*A. chroococcum* and *B. megaterium* were grown separately, wherein the first strain was grown in 300 mL of growth medium consisting of 10g peptone, 10 g dextrose and 5g NaCl per 1L of distilled water, while the latter was grown in 300 mL tryptone soya broth. Both strains were incubated with mixing on a rotary shaker, for example at 150 rpm, and at temperature around 30 °C for 72 h and 48 h, respectively.

Afterwards, they were separately encapsulated with the process comprising the following steps:

- mixing of the whole medium with bacterial cells with alginate, starch and optionally a colorant,
- dropping the mixture from the previous step into calcium chloride using a nozzle with 8 holes, while encapsulation mixture is pumped using mechanical pump,
- incubation for about 15 min of the mixture obtained in the previous step to allow gelation and to obtain encapsulates;
- rinsing the encapsulates with water to remove surface components,
- spreading the rinsed encapsulates onto a drying pan and drying in an oven at 37 °C for 2 days.

**Example 3: Preparation of the biofertilizer**

The obtained encapsulates may be used alone or in combination, wherein both bacterial strains are preferably combined at a ratio 1:1, however, the ratio could also be any other. Also, any other plant-promoting bacterium may be used together with any of the *A. chroococcum* or *B. megaterium* encapsulates, such as other species belonging to genera *Bacillus, Pseudomonas* and *Azotobacter.* Further components known in the field of biofertilizer preparation may be added to the biofertilizer according to this example, but they are not needed. The preferred embodiment comprises only encapsulated bacterial cells and due to the improved encapsulation method also their growth media, metabolites and other possible compounds present in used growth media.

**Example 4: Survival of encapsulated strains**

Survival of bacterial strains was determined immediately after encapsulation, as well as after two months of storage at room temperature. 10 g of the encapsulated samples were diluted in 9 ml of 1.5% Na-citrate (Na-citrate was used to enable rapid solubilisation of the particles). Afterwards, serial decimal dilutions in 0.9% NaCl were prepared. The dilutions obtained of *Azotobacter* encapsulates were transferred to Fiodorov's medium, and Nutrient Agar was used for *Bacillus* encapsulates. Incubation at 30 °C followed by enumeration of colonies with specific morphology was used to determine the viability of bacteria.

The results presented in table 1 suggest that roughly the same number of cells was present in liquid media prior encapsulation and after encapsulation. More importantly the number of viable cells in encapsulates stored for two months has not dropped in comparison to freshly prepared encapsulates, wherein there was almost no drop in cell number of *A. chroococcum,* whereas the number of viable *B. megaterium* cells in encapsulates dropped from $8.40*10^7$ to $4.90*10^7$. This result, however, suggests that majority of cells is still able to be active upon transfer to a nutrient-rich medium such as soil. Hence, both tested bacterial strains exhibited suitable survival and the encapsulation method is thus suitable for preparation of stable biofertilizers. The technique of encapsulation with starch and simple drying at 37 °C do not have the disadvantages observed with spray drying technique, wherein the prepared biofertilizer can be stored much longer at room temperature in simple packages. The granulation of the biofertilizer prepared with the method according to example 2 enables use already available mechanization.

Table 1: Survival of encapsulated strains.

| Strain | CFU/ml in liquid culture | CFU/g after encapsulation | CFU/g after 2 months of storage at room temperature |
|---|---|---|---|
| *B. megaterium* 11/3 | $1.1*10^8$ | $8.40*10^7$ | $4.90*10^7$ |
| *A. chroococcum* F 14/2 | $7.9*10^8$ | $7.06*10^8$ | $7.00*10^8$ |

**Example 5: Results of field testing of the prepared encapsulates on germination and early growth parameters for sugar beet treated with encapsulates and grown for four weeks in commercial substrate**

Seeds of sugar beet were inoculated with bacterial strains, previously encapsulated with alginate as carrier using spray-drying technique. One of the alternative possibilities to encapsulate the same strains as in example 2 is to use spray drying and alginate as a carrier. However, the biofertilizer prepared with spray drying technique has a disadvantage of viability loss during storage. So prepared encapsulates can be stored at room temperature only if they are in some kind of hermetically sealed package. Otherwise, decreased viability as well as changes in visual appearance of encapsulates occurs.

The protocol was: 10 g/100 ml of liquid maltodextrin as cell carrier was added to the cell suspension (i.e. whole cell culture medium with suspended cells) before the encapsulation process, wherein maltodextrin was dissolved on the mixer for 30 min followed by spray drying with conditions: inlet temperature: 130°C; outlet temperature: ~65°C; liquid flow: 8 ml/min.

Three portions of 50g of sugar beet seeds were coated with 1ml of glycerol. Two portions were than coated with 2g of one of the powdered encapsulated cultures, and one was treated with 2g of consortium mixture in ratio 1:1. 30 seeds were used and planted in the commercially available substrate (Floragard®, Germany). Sugar beet was grown for four weeks at room temperature, and regularly watered to ensure good development of seedlings.

Table 2: Germination, fresh biomass and dry weight of sugar beet seeds treated with *B. megaterium, A. chroococcum* and consortium of *B. megaterium* and *A. chroococcum*

| | Control | *B. megaterium* 11/3 | *A. chroococcum* F14/2 | Consortium |
|---|---|---|---|---|
| Number of germinated seeds (max. 30) | 28 | 30 | 29 | 29 |

(continued)

|  | Control | *B. megaterium* 11/3 | *A. chroococcum* F14/2 | Consortium |
|---|---|---|---|---|
| % of emergence in substrate | 93,3 | 100 | 96,7 | 96,7 |
| Average fresh biomass (g) | 1,64 | 1,86 | 1,19 | 1,48 |
| Average dry weight (g) | 0,12 | 0,18 | 0,12 | 0,15 |

As can be seen from Table 2, the number of germinated seeds is quite similar if compared to control, while the percent of emerged seeds in the substrate is higher in seeds treated with any of the fertilizers comprising either *B. megaterium,* either *A. chroococcum* or the combination of both. Especially presence of *B. megaterium* seems to improve average fresh biomass and average dry weight.

**Example 6: The effect of the biofertilizer on apple Golden Delicious yield**

An apple orchard was divided into two fields: a control field and a treatment field to which the biofertilizer was applied. Both fields were subjected to the same agricultural management practice - whole orchard was treated with 200kg/ha of mineral fertilizer NPK 14:7:16 . 800g/ha of the biofertilizer mixture was applied in the treatment field. The mixture consisted of encapsulated bacterial strains *B. megaterium* and *A. chroococcum* prepared as described above in ratio 1:1. The encapsulated biofertilizer was applied when the soil temperature reached 12°C and air temperature was above 15°C (at the beginning of April). The effect of the biofertilizer according to the invention had been applied two years in a row and the results as means and standard error are shown in the table below.

Table 3: The effect of the biofertilizer according to the invention comprising *B. megaterium* and *A. chroococcum* on apple Golden Delicious yield

| Treatments | Year | Yield per tree (kg) | Fruit mass (g) | Fruit diameter (cm) | Yield (kg/ha) |
|---|---|---|---|---|---|
| Control | 2017 | 8.60+/-0.50* | 210.4+/- 7.7 | 8.04+/- 0.37 | 29.799+/- 1.542 |
| | 2018 | 15.30+/-0.51 | 196.1+/- 5.0 | 7.47+/- 0.43 | 53.014+/- 2.087 |
| Treated with encapsulated bacteria | 2017 | 9.26+/-0,20 | 209,7+/- 4.4 | 8.46+/- 0.15 | 32.085+/- 1.263 |
| | 2018 | 33,00+/-0.91 | 286.8+/- 2.6 | 8.97+/- 0.14 | 114.345+/- 2.330 |

ANOVA was used as the statistical test (software Statistica (StatSoft, Tulsa, OK, USA)). Mean values of data were compared by Tukey test at significance level of $p$=0.05. The results showed that all of the parameters determined in the first year in the treated field were not significantly different from control conditions. On the other hand, results from the next year show statistically significant difference between values of all parameters in treatment field and in control conditions. The yield was higher, the apples larger and their mass increased as well.

Also the content of macro and microelements in apples treated with the biofertilizer in this experiment was determined by desintegration of samples in acids (70% $HNO_3$, 65% $HclO_4$, and 70% $H_2SO_4$; 5:1:1), digestion and filtration, as described in Allen et al. (1986). The dillution of basic solution 1/100ml was used for determination of macroelements. The content of macroelements was determined by atomic absorption spectrometer (Perkin-Elmer PinAAcle 500, USA)

Table 4: content of macro and microelements in apples treated with the biofertilizer according to the invention comprising *B. megaterium* and *A. chroococcum*

| Treatments | year | P | K | Ca | Mg | Mn | Co | Ni | Pb |
|---|---|---|---|---|---|---|---|---|---|
| | | % | | | | mg kg$^{-1}$ | | | |
| Control | 2017 | 0,06 | 0,40 | 0,02 | 0,02 | 1,47 | 0,25 | 0,11 | 0,83 |
| | 2018 | 0,04 | 0,40 | 0,04 | 0,02 | 1,48 | 0,35 | 0,15 | 0,75 |
| Treated with encapsulated bacteria | 2017 | 0,07 | 0,51 | 0,05 | 0,03 | 1,98 | 0,46 | 0,28 | 0,96 |
| | 2018 | 0,08 | 0,61 | 0,05 | 0,02 | 2,12 | 0,49 | 0,25 | 0,96 |

The content of P, K, Mn, Co, Ni and Pb increased in the apples treated with the biofertilizer according to the invention as described in example 6, while Ca and Mg levels remained similar to control apples. Presented results suggest that the biofertilizer improves the yield of apples, as well as their mass, size and element content, wherein the effect is pronounced after consecutive treatments (i.e. consecutive years). Hence, the biofertilizer seems suitable for agricultural use and for treatment of fruit trees.

**Example 7: The effect of the biofertilizer according to the invention on corn yield**

The effect of the biofertiliser according to the invention comprising *A. chroococcum* and *B. megaterium* in the ratio 1:1 as described above has been tested in the corn field. The biofertilizer has been applied in amount 800g/ha to corn fields, wherein the corn cultivars were Hibrid Pionir 9241 and Hibrid DKC 4717. The biofertilizer was applied once in the period of sowing. The control fields were remained untreated. The final yield of corn growing was weighed and calculated with regard to the field size. The yield of corn cultivar Hibrid Pionir 9241 in the control field was 12620 kg/ha, while the treated field had a yield of 12978. A similar ratio has been observed for the corn cultivar Hibrid DKC 4717, wherein the control yield was 12800 kg/ha and the yield of the treated field was 13350. These results imply that the biofertilizer improves the yield of corn and further confirms its suitability for use in agriculture.

**Claims**

1.    A bacterial strain for use in a biofertilizer, **characterized in that** it simultaneously exhibits:

- production of phosphatases for mineralization of organic phosphorus compounds, and
- phosphorus solubilisation for solubilizing insoluble phosphorus salts.

2.    The bacterial strain according to claim 1, **characterized in that** it is selected from the group of bacterial strains belonging to genus *Azotobacter* or *Bacillus*.

3.    The bacterial strain according to claim 2, **characterized in that** it is *Azotobacter chroococcum* LMG P-31444.

4.    The bacterial strain according to claim 2, **characterized in that** it is *Bacillus megaterium* LMG P-31445.

5.    Encapsulation method, which comprises the following steps:

a) growth of at least one bacterial strain of plant-growth promoting in a suitable medium,
b) mixing of the whole medium from step a) with alginate, starch and optionally a colorant,
c) dropping the mixture from step b) into calcium chloride;
d) incubation of the mixture obtained in step c) to allow gelation, preferably for 15 to 30 minutes, more preferably for about 15 min, to obtain encapsulates;
e) optionally rinsing the encapsulates obtained in step d) with water to remove surface components,
f) spreading the rinsed encapsulates onto a suitable surface such as a drying pan and drying at temperature at least 30 °C for at least 2 days or for less if the temperature is higher; wherein the preferred drying is preferably in an oven at 37 °C for 1 to 2 days.

6.    The encapsulation method according to the preceding claim, **characterized in that** step c is preferably performed using a nozzle with 8 holes, while encapsulation mixture is pumped using mechanical pump, or other techniques such as vibration technique, electrostatic extrusion, and dispersion with compressed gas.

7. The encapsulation method according to any claim from 5 to 6, **characterized in that** bacterial strain for encapsulation may be one or more, preferably selected from the group of bacterial genera: *Bacillus, Azotobacter,* and/or *Pseudomonas,* more preferably *B. megaterium* and/or *A. chroococcum,* wherein strains may be grown and encapsulated separately or grown and/or encapsulated together, depending on strain growth requirements.

8. The encapsulation method according to any claim from 5 to 7, **characterized in that** *A. chroococcum* and *B. megaterium* are grown separately, wherein the first is preferably grown in peptone, dextrose and NaCl comprising medium, while the latter is preferably grown in tryptone soya broth.

9. The encapsulation method according to any claim from 5 to 8, **characterized in that** the preferred ratios in step b) of the method described above are:

   - at least 1:1, preferably 2:1 liquid bacterial culture : alginate solution; wherein the alginate solution is preferably 3% (w/w);
   - 5 to 15 g, preferably 10g of starch per 100ml of liquid bacterial culture depending on required size of encapsulates; and
   - the amount of the optional colorant is 0.2g/100ml of the encapsulation mixture.

10. Encapsulated bacterial cells prepared by the method according to any claim from 5 to 9, wherein encapsulated bacterial cells comprise components of cells and bacterial growth medium and/or cell metabolites and/or molecules present in used growth medium.

11. Encapsulated bacterial cells according to the preceding claim, **characterized in that** the bacterial cells are selected from the group of bacterial genera: *Bacillus, Azotobacter,* and/or *Pseudomonas.*

12. A bio-fertilizer comprising encapsulated bacterial cells according to claim 11.

13. The bio-fertilizer according to the preceding claim, **characterized in that** it comprises *A. chroococcum* LMG P-31444 and *B. megaterium* LMG P-31445 in ration 1:1.

14. Use of the bacterial strain according to any claim from 1 to 4, encapsulated bacterial cells according to any claim from 10 to 11 and/or the biofertilizer according to any claim from 12 to 13 in agriculture for increasing phosphorus availability for plants.

Europäisches
Patentamt

European
Patent Office

Office européen
des brevets

# EUROPEAN SEARCH REPORT

Application Number

EP 20 16 8415

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAHIM NOSRATI ET AL: "Phosphate solubilization characteristics of efficient nitrogen fixing soil Azotobacter strains", IRANIAN JOURNAL OF MICROBIOLOGY, vol. 6, no. 4, 1 August 2014 (2014-08-01), pages 285-295, XP055727854, | 1-3,14 | INV. C12N1/20 C12N1/04 A01N63/20 A01N63/22 C05F11/08 |
| A | * the whole document * | 13 | ADD. C12R1/065 |
| A | ROI A A ET AL: "Biological Properties of the Phosphorus-Mobilizing Bacillus subtilis Strain IMV V-7023", APPLIED BIOCHEMISTRY AND MICROBIOLOGY, KLUWER ACADEMIC PUBLISHERS-PLENUM PUBLISHERS, NE, vol. 40, no. 5, 1 September 2004 (2004-09-01), pages 476-481, XP019293732, ISSN: 1608-3024 * the whole document * | 1-3,13, 14 | C12R1/11 |
| A | CN 104 498 403 A (BEIJING ACAD AGRIC & FORESTRY) 8 April 2015 (2015-04-08) * the whole document * | 1-3,13, 14 | TECHNICAL FIELDS SEARCHED (IPC) C12N C12R A01N |
| A | CN 107 841 480 A (GUANGZHOU JUNLUO BIOTECHNOLOGY CO LTD) 27 March 2018 (2018-03-27) * the whole document * | 1-3,13, 14 | C05G C05F |

-/--

~~The present search report has been drawn up for all claims~~

| Place of search | Date of completion of the search | Examiner |
|---|---|---|
| The Hague | 11 September 2020 | Lejeune, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 20 16 8415

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MOHAMMAD ALI MALBOOBI ET AL: "Solubilization of organic and inorganic phosphates by three highly efficient soil bacterial isolates", WORLD JOURNAL OF MICROBIOLOGY AND BIOTECHNOLOGY, KLUWER ACADEMIC PUBLISHERS, DO, vol. 25, no. 8, 18 April 2009 (2009-04-18), pages 1471-1477, XP019734626, ISSN: 1573-0972, DOI: 10.1007/S11274-009-0037-Z * the whole document * ----- | 1-3,13,14 | |
| A | LIU LILI ET AL: "Development of an Engineered Soil Bacterium Enabling to Convert Both Insoluble Inorganic and Organic Phosphate into Plant Available Phosphate and Its Use as a Biofertilizer", MOLECULAR BIOTECHNOLOGY, HUMANA PRESS, BOSTON, vol. 57, no. 5, 14 January 2015 (2015-01-14), pages 419-429, XP035485396, ISSN: 1073-6085, DOI: 10.1007/S12033-014-9834-1 [retrieved on 2015-01-14] ----- | 1-3,13,14 | |

TECHNICAL FIELDS
SEARCHED (IPC)

~~The present search report has been drawn up for all claims~~

| Place of search | Date of completion of the search | Examiner |
|---|---|---|
| The Hague | 11 September 2020 | Lejeune, Robert |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

&  : member of the same patent family, corresponding
  document

EPO FORM 1503 03.82 (P04C01)

Europäisches
Patentamt

European
Patent Office

Office européen
des brevets

Application Number

EP 20 16 8415

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

3(completely); 1, 2, 13, 14(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
### SHEET B

Application Number

EP 20 16 8415

The Search Division considers that the present European patent application does not comply with the requirements of unity of invention and relates to several inventions or groups of inventions, namely:

1. claims: 3(completely); 1, 2, 13, 14(partially)

   A bacterial strain exhibiting simultaneously production of phosphatases for mineralisation of organic phosphorous compounds and solubilisation of insoluble phosphorous salts; in particular the strain Azotobacter chroococcum LMG P-31444
   ---

2. claims: 4(completely); 1, 2, 13, 14(partially)

   The strain Bacillus megaterium LMG P-31445
   ---

3. claims: 5-12(completely); 13, 14(partially)

   Encapsulation method of a plant-growth promoting bacterial strain; encapsulated bacterial cells and biofertilizer comprising said encapsulated cells.
   ---

## ANNEX TO THE EUROPEAN SEARCH REPORT
## ON EUROPEAN PATENT APPLICATION NO.

EP 20 16 8415

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2020

| Patent document cited in search report | Publication date | Patent family member(s) | Publication date |
|---|---|---|---|
| CN 104498403 A | 08-04-2015 | NONE | |
| CN 107841480 A | 27-03-2018 | NONE | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 108753654
- FR 2806420
- CN 105967856
- CN 110438036
- RU 2008129928
- EP 2227931 A
- SU 1703634
- CN 102765983
- CN 104961591
- CN 109206204
- CN 101244957

**Non-patent literature cited in the description**

- **ZANJANI et al.** *Iran J Pharm Res.,* 2014, vol. 13 (3), 843-852
- BCCM Belgian Coordinated Collections of Microorganisms. *BCCM/LGM Bacteria Collection,* 28 May 2019